# EUROPEAN PATENT SPECIFICATION

(11) **EP 2 450 073 B1**
(45) Date of publication and mention of the grant of the patent: **08.11.2017**
(21) Application number: 10793699.9
(22) Date of filing: 02.07.2010
(51) Int. Cl.: A61M 16/00, A61H 31/00, G05D 16/04, A61M 16/10, A61M 16/06

(54) **COMPACT DEVICE FOR CONTROLLING AND MODIFYING THE PRESSURE OF A GAS OR A MIXTURE OF GASES**
KOMPAKTE VORRICHTUNG ZUR STEUERUNG UND MODIFIKATION DES DRUCKS EINES GASES ODER EINER GASMISCHUNG
DISPOSITIF COMPACT POUR RÉGULER ET MODIFIER LA PRESSION D'UN GAZ OU D'UN MÉLANGE DE GAZ

(30) Priority: 02.07.2009 CO 09068500
(43) Date of publication of application: 09.05.2012
(73) Proprietor: Fundacion Universidad Del Norte, Atlántico (CO); Sociedad Biotecnologia Y Bioingenieria Core S.A., Barranquilla (CO)
(72) Inventor: GUTIERREZ FONSECA, Jaime Eduardo, Barranquilla (CO); OVALLE OREJARENA, Oscar Omar, Barranquilla (CO); BULA SILVERA, Antonio, Barranquilla (CO)
(74) Representative: ABG Patentes, S.L.
(86) International application number: PCT/IB2010/001642
(87) International publication number: WO 2011/001277

(56) References cited:
- WO-A1-81/00212
- CN-Y- 2 877 741
- DE-A1- 2 929 996
- ES-A1- 2 319 832
- ES-T3- 2 262 476
- JP-U- S5 427 574
- US-A- 3 827 433
- US-A- 3 869 771
- US-A- 5 271 391
- US-A1- 2005 072 470
- US-A1- 2005 072 470
- DATABASE WPI Week 201036, Derwent Publications Ltd., London, GB; AN 2010-F57711, XP003027062 & CN 2 877 741 Y (QINGDAO SHI HUANGDAO QU ZHONG) 14 March 2007

## Description

### 1. Field of the invention.

This invention relates to controlling gas pressure in a circuit, by generating bubble through a liquid, when this effect is used to produce a continuous positive airway pressure.

### 2. Description of the art.

The present invention relates to gas pressure control through devices that are designed to maintain a stable pressure in patients with respiratory failure, specifically in newborns or infants. For this, continuous distending pressure defined as a positive gas pressure across the airway or sufficient enough to keep the alveoli open, must be considered, which is held during the natural cycle of breathing. The natural cycle consists of inspiration, exchange and expiration. When this phenomenon occurs while the patient breathes spontaneously, is called CPAP (Continuous Positive Airway Pressure) and when it occurs during the application of mechanical ventilation (connected to a mechanical ventilator) it is called PEEP (Positive end-expiratory Pressure), since positive pressure is applied at the end of expiration. This method is used to increase pressure in the lungs above atmospheric pressure.

PEEP and CPAP are used to correct respiratory failure and atelectasis (collapse and consequent dysfunction of the lungs), or when there is decreased functional residual capacity (percentage of lung capacity with the lungs above the minimum necessary to survive), or alteration of ventilation perfusion (alteration in the amount of gas reaching the alveoli and blood supply reaching the lungs) or pulmonary edema (fluid accumulation in lung tissue that hinders gas exchange).

The application of continuous positive airway pressure for the management of hypoxia (low blood oxygen level) is one of the great breakthroughs in neonatal respiratory therapy. This application improves oxygenation therapy by an increase in functional residual capacity, increases lung tissue area capable of gas exchange, through the recruitment of alveoli (opening air sacs that are closed). This reduces the imbalance between pulmonary perfusion and gas exchange (*intra pulmonary shunt).* It also reduces the need to force the lungs to full capacity (functional capacity plus residual), which is beneficial for the patient, since forcing the lungs produces an increase in air entering the lungs.

The technique by which CPAP is delivered to the nasal airway (NCPAP) is widely known and used in preterm and low birth weight newborns as a preventive measure of respiratory distress syndrome and the need for intubation and mechanical ventilation¹.
¹ "2005 American Heart Association (AHA) Cardiopulmonary Resuscitation Guidelines for (CPR) and Emergency Cardiovascular Care (ECC) of Pediatric and Neonatal Patients: Neonatal Resuscitation Guidelines." Pediatrics, Official Journal of the American Academy of Pediatrics. ISSN 0031-4005, August 28, 2006.

The general mechanism requires placing a mask over your nose and mouth, or some "prongs" that are inserted directly into the nostrils of the baby. Either of these two elements are connected to a standard ventilator circuit. The ventilation circuit must maintain a flow of gas pressure above atmospheric pressure by applying resistance to the gas outlet end of the circuit (for example, a water level). This increased pressure forces the gas out of the prongs or mask into the infant's airway. The degree of ventilatory support given is proportional to the pressure in the ventilation circuit, measured in centimeters of water. There are no known systems that measure the ventilatory support in terms of gas volume passing through the circuit, or the gas pressure in the mask, prongs or lungs.

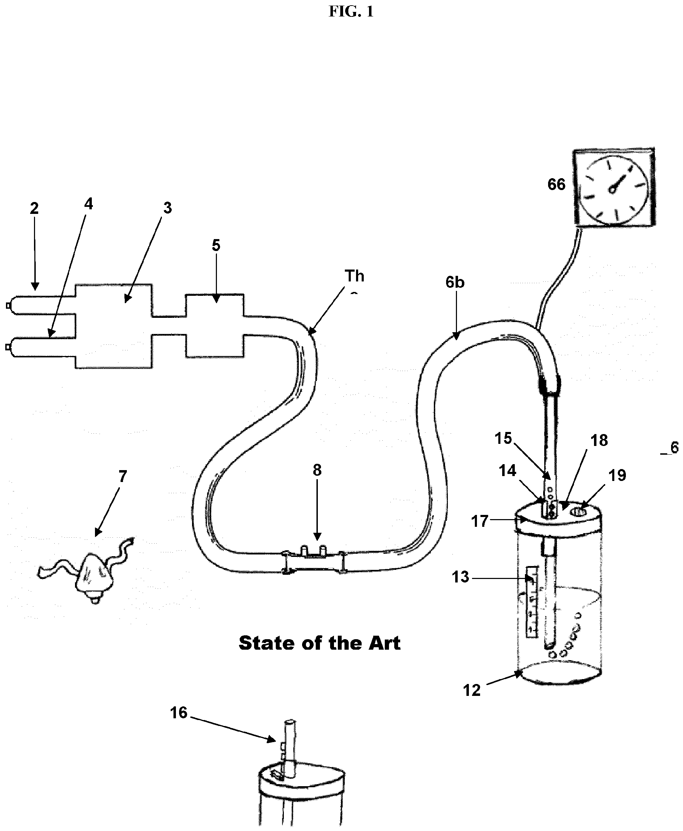

There are two known traditional ways of applying NCPAP. The first is the ventilatory circuit connecting to a ventilator machine, which is scheduled for not cycling, but to provide a continuous positive airway pressure, which the dose is programmed into the machine and is measured by a manometer. This mechanism is effective, but use means wear a team of mechanical ventilation, and blocks for other patients who may require higher priority. The other method is through the "bubble CPAP", of which the schematic diagram shown in Figure 1 is a typical example. In the diagram depicts an oxygen source 1 (not shown) from which a connection through conventional 2 gas is supplied to a mixing chamber 3. Through another entry 4 is supplied with air the camera. The oxygen and air are mixed in the chamber and flows through a conventional humidifier 5, in which the gas mixture is humidified to the required moisture. The initial part of a conventional ventilation tube 6a is connected to the output of the humidifier. The final part of the first tube is connected to a device that creates a pressure differential to the patient (this may be a conventional face mask) 7, or with a conventional nasal prongs 8. Whether 7 face mask, nasal prongs or 8, are connected in turn with the initial part of another conventional ventilation tube 6b, whose other free end is immersed in a liquid 9, or connected to the leading edge of some kind rigid tube 10 whose other end is immersed in a liquid, which may be water, saline, dextrose in distilled water or other liquid in a receptacle 12. This receptacle can have a top 17, with holes for inlet 18, or the air outlet 19. This causes the gas mixture to flow from the humidifier, the vent pipe 6a to 7 facemask, nasal prongs or 8 and then into the lungs of the newborn. The inhaled air is passed to the second vent pipe 6b and out the open and submerged end of the vent pipe 6b, or the free end of the rigid tube and submerged 10 where the gas mixture 11 forms bubbles that escape to the surface of liquid 9 and finally dissolve into the environment. In this system, the depth to which you are immersed either the free end of the rigid tube 10, on the surface of the liquid in the receptacle, determines the level of pressure in the ventilator circuit 6, and therefore in the nasal prongs or face mask 8 7, which in turn determines the level of ventilatory support that is supplied to the newborn. This level is measured in centimeters, usually printed on a scale 13, either drawn or attached to the wall of the receptacle, or rigid tube that is half submerged in the liquid, so that the position of the submerged end of the tube ventilation, can be read in reference to this scale. Typically, the pressure in the ventilation circuit 6 can vary between 1 to 10 inches water by adjusting the depth of the bubble point to the surface of the liquid 9. That is, the liquid level is static and what varies is the tube. Normally after you have placed the tail end of the ventilatory circuit at the desired depth, we proceed to fix it by some auxiliary means such as guide tubes 14, 15 slots, extensions or wedges 16. Additionally, you can connect a 66 gauge to measure pressure within the ventilatory circuit 6. However, the means by amending the depth of immersion of the end of the ventilatory circuit in the liquid is unstable. Even in those versions that have locks, extensions, or wedges, resistance does not guarantee the same level of immersion that is preserved and may be undesirable changes in the level of ventilatory support delivered to the patient.

Additionally, existing systems are not suitable for use in transportation, as the liquid level changes greatly with the movement of a vehicle, and may even pour the liquid from the receptacle, resulting in pressure loss and disruption of the therapy. This is very relevant because the main users of bubble CPAP therapy are low birthweight babies, of which a large part born far from the high-level care facilities, requiring to be transported by ambulance, time during which CPAP therapy is Salvatori. It thus requires the provision of a CPAP device that can be used safely for transportation in vehicles.

### 3. Brief description of the graphics.

The invention will be described by the following drawings in which reference numbers are included to identify the constituent parts:
**Figure 1** is a circuit schematic diagram of a conventional bubble CPAP devices known in the art.
**Figure 2** Figure is a schematic of one embodiment of the invention where the device is called nasal prongs connected to a power and gas.
**Figure 3** Figure is a schematic of another embodiment of the invention where the device of the present invention is connected to a humidifier, heater and a nasal prongs to the source of gas.
**Figure 4** Figure is a schematic of another embodiment of the invention where the device of the present invention recycles the gas system.
**Figure 5** is a schematic front isometric view of a preferred embodiment of the invention.
**Figure 6** is a view of the upper surface of the lid of the apparatus shown in Figure 5.
**Figure 7** is an isometric view of the bottom of the apparatus shown in Figure 5.
**Figure 8** is an isometric view of the back of the cover attached to the inner vessel for heating and humidification.
**Figure 9** is a schematic isometric view of the internal elements of the cover of the apparatus shown in Figure 5.
**Figure 10** is an extension of the stabilization mechanism of the liquid level in a preferred embodiment of the present invention.

### 4. Brief description of the invention.

The invention is defined by the appended claims. The present disclosure consists of a device for controlling and modifying the pressure of a gas or a mixture thereof, within a system in which the pressure and changes thereof are generated by gas bubble through a liquid but, unlike prior art, pressure varies changing the liquid level.

In a preferred example, the device comprises a liquid inlet hose with a shutoff valve, a receptacle, a lid, a fixed bubble tube and overflow tube. The inlet hose allows adjusting the liquid level in the receptacle, which varies so the amount of liquid over the bubble point, and so the pressure generated. In another preferred embodiment, the device also has an element of stabilization of the liquid level comprising a screw and double perforated platform, which can move over the length of the screw, a fluid outlet hose with a shutoff valve and an internal humidification and heating receptacle. This system prevents the liquid level from varying due to sudden movements such as inside a moving vehicle.

Unlike prior art, the present disclosure provides a modification of ventilatory support by means of varying the liquid level in the receptacle and not by varying the bubble tube height. This provides improved safety for controlling respiratory therapy in patients. Additionally, the present invention provides the possibility that the device may be used in moving vehicles using level stabilizers that prevent the variation of the respiratory system pressure due to movement of the device.

### 5. Detailed description

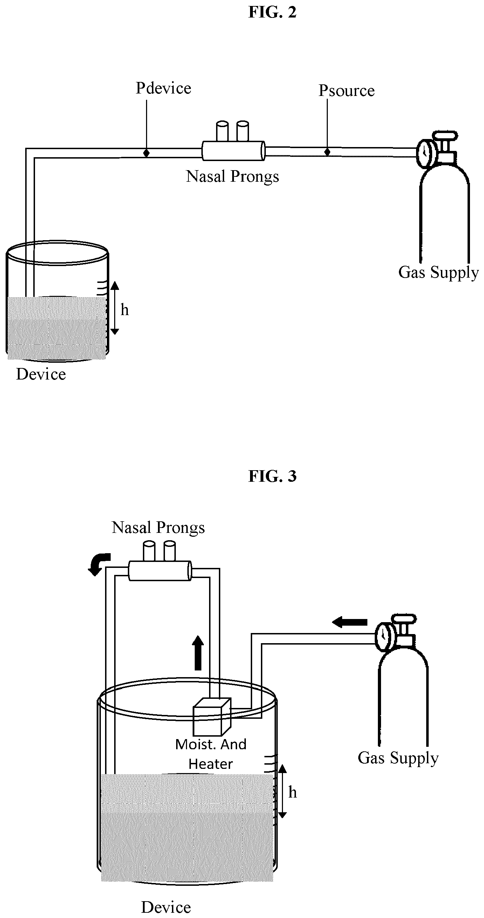

Referring to Figure 2, a scheme is shown of the airflow that is generated when connecting an embodiment of the present disclosure to a respiratory tool for a patient connected to a gas source (Fig. 2 in this tool are breathing nasal prongs, but may also face masks, endotracheal tubes or other delivery device). A gas source may include a single gas or mixture of two or more gases used in hospital care or treatment to patients that require breathing assistance. The outlet pressure of gas flows at a given pressure (Pfuente) to the nasal prongs. In the phase of expiration of the patient, the pressure of the liquid to the gas (Pdispositivo), which is the same that is exerted on the patient's lungs is slightly higher than atmospheric pressure (this value depends on the amount of volume of liquid in the receptacle there is the device of the present invention, the liquid level is represented as h-). Thus, the pressure exerted by the device on the lungs (Pdispositivo) does not allow the patient's lungs from collapsing.

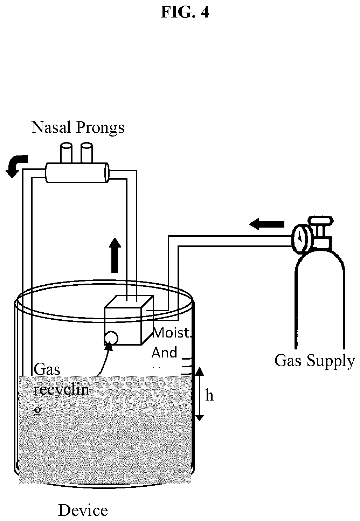

In a most preferred embodiment of the present disclosure illustrated in Figure 3 shows an additional humidification and heating device incorporated into the receptacle. This Figure shows the flow of air that is generated when the humidifier and heater device is present. In this mode, the air flow is similar to that shown in Figure 2, but with the difference that the flow of air through nasal prongs is by pre-heated and humidified to a predetermined level (established in various treatment protocols) . Here, the pressure generated in the prongs is just as controlled by the level of liquid in the receptacle of the device of the present disclosure. Figure 4 shows the schema to another preferred embodiment, similar to that shown in Figure 3, but with the property that the gas bubble through the liquid in the pan, return to the ventilatory circuit through an orifice or venturi on the device humidifier and heater. The details of the operation of these schemes are described in detail in the description of the figures below.

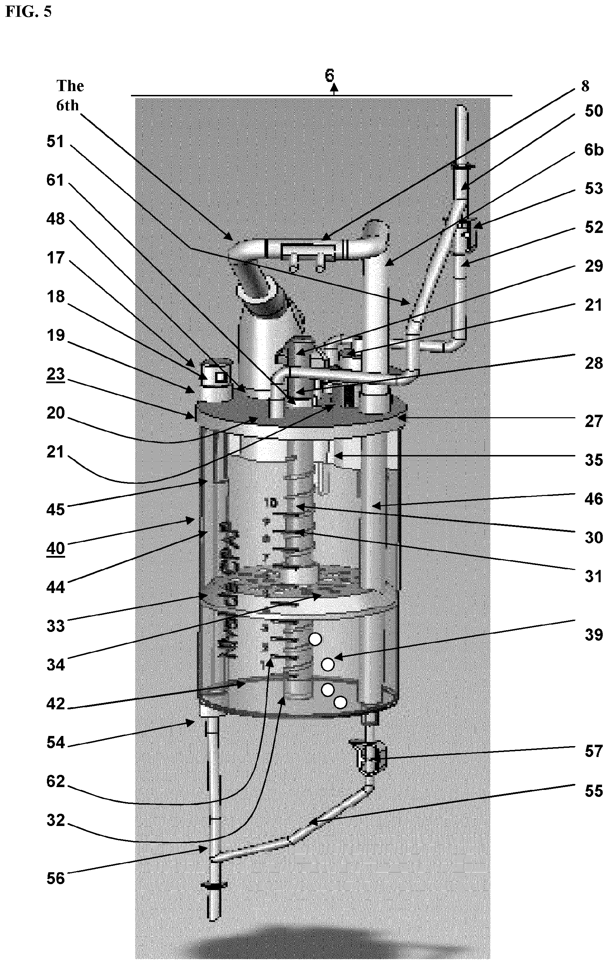

Figure 5 shows the preferred embodiment of the present disclosure schematically in Figure 3, in which the device is formed by a liquid inlet hose 50 with a bypass valve 53, a receptacle 40, a cover 23, a tube of bubble 56, an overflow tube 44 and stabilization of pressure, a device to stabilize the liquid level consists of a rotating shaft 30 and a perforated platform 33, a fluid outlet hose 55 with shut-off valve 57, an inner receptacle humidification and heating 35, conventional connection pipes are connected to the holes in the lid of the device and nasal prongs 8.

The device of the present disclosure is connected to a fluid source (not shown) via a hose 50. This hose is divided into two branches, the fill hose 52 of the inner receptacle and heating humidifier 35 and the increased level hose 51, which has a closure valve 53. The far end of the filling hose 52 is connected with the hole 20 of the cover 23, which connects the inner vessel 35. The far end of the hose 51 level increase is connected to port 26 of the top 23.

The upper end of overflow tube 44 and stabilization of pressure is connected to the pressurizing hole 19 located on the underside of the lid 23. The lower end of the overflow pipe 44 is connected to the overflow hole 60 located on the bottom of the receptacle 40. The overflow pipe 44 has the overflow hole 45 which is located at an elevation of the bottom of the receptacle 40, which allows, in any case, the level of liquid overflow. In a preferred embodiment, this height is 10 inches tall.

The device shown in Figure 5 is connected to a ventilator circuit where the gas source is connected through a vent hose connector for conventional gas sources 21 hospitals located in the top 23. The hole 48 is connected to the end of the 6th vent hose which is connected to the nasal prongs 8. The other end of the prongs 8 is connected to a vent hose that connects to 6b hole 25 of the top 23. At the end of the absorber 46 is a bubble hole 47, which bubbles out 39. As the absorber 46 is fixed, provided the level of CPAP is set by changing the level of liquid 42 within the receptacle 40. Thus, the higher level of liquid, bubble hole 47 is submerged deeper, in turn generating higher levels of CPAP.

The receptacle 40 shown in Figure 5 is made of conventional materials such as glass or plastic, made by injection or machining.

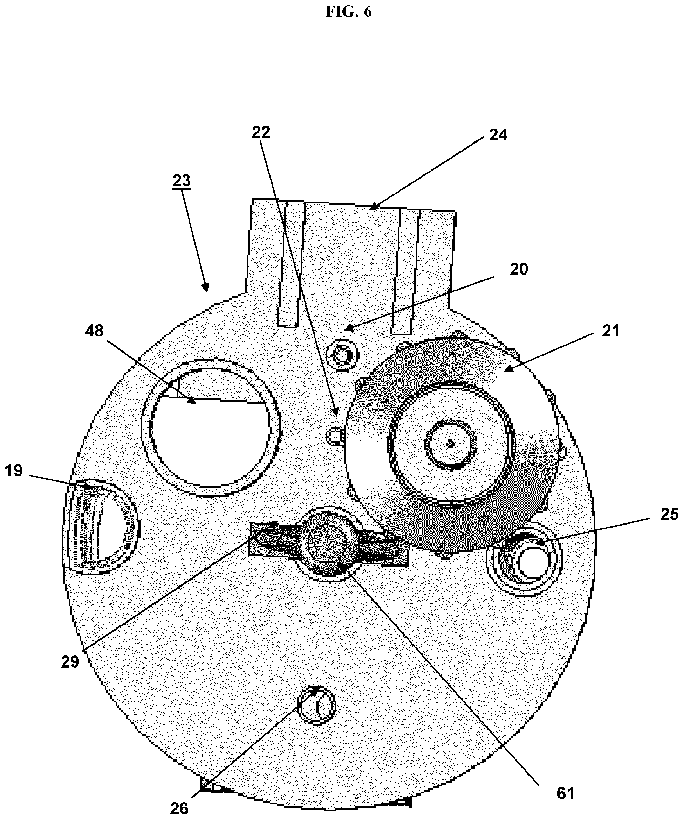

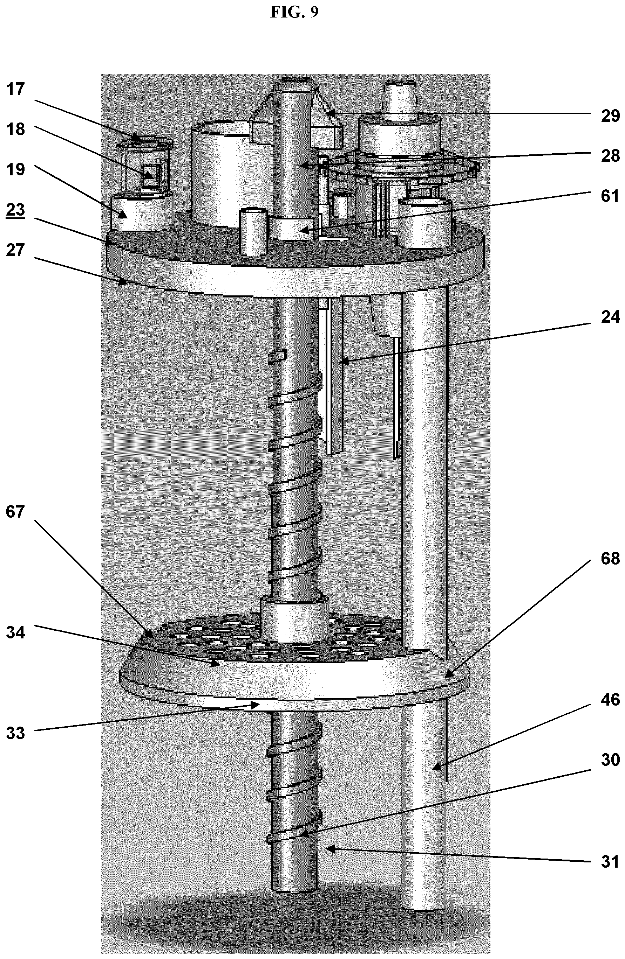

Figure 6 shows a view of the upper cover 23, which is screwed into the top opening of receptacle 40. At the top of the lid 23 is pressurizing hole 19, which ensures that when the receptacle 40 is closed with the lid 23, the pressure on the surface of the liquid in the receptacle 40 is equal to atmospheric pressure. On this hole 19 may be located pressurizing a security cap 17 (see Figure 9), which has lateral openings 18 (see Figure 9), whose function is to prevent the entry of smaller solids into the receptacle 40. The cover 23 also has a hole 26 for connecting the hose to increase liquid level 50 which is where you fill the receptacle 40. The lid also has an anchor hole 61 to the rotating shaft 30 that is part anchored stabilizer level 33. In the embodiment shown in Figure 6, the hole 61 is in the center of the lid 23. The cover additionally has a hole 25 which connects the end of conventional ventilation tube is connected to one end of the prongs 8 or gas mask 7. The edge of the lid has a support stand conventional hospital 24, which serves to secure the device to a hospital or ambulance stand. The lid has a hole 20 for the entry of liquid into the receptacle internal humidification and warming 35. The lid also has a hole 22 for insertion of an electrical resistance 36 in the inner vessel 35. The cover 23 has a conventional connector hospital gas source 21, which has a gas mixing venturi, which as already explained, the air gas mixture with the gas entering the gas source. The cover further has a hole 48 for connecting the front end of the vent pipe that is connected to one end of the prongs 8 or gas mask 7 with the inner vessel 35.

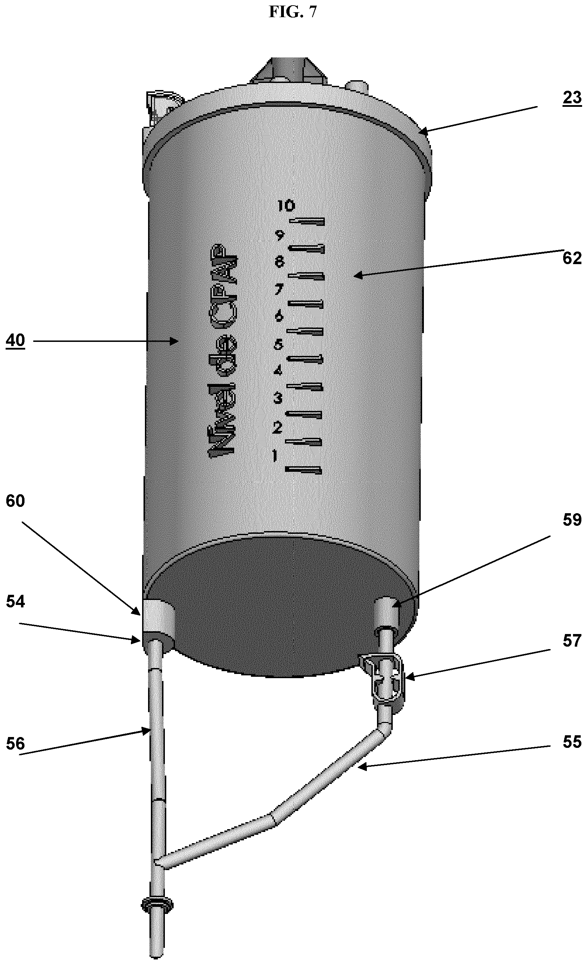

Figure 7 shows the bottom of the receptacle 40 which has an outlet 60 which is connected to the overflow pipe 44. The bottom of the receptacle 40 also has a hole 59 which connects the hose to reduce the liquid level 55.

As illustrated in Figure 7, the device has a bottom drain system consists of an overflow tube 54, whose first end is connected to the outlet 60 in the outer face of the bottom of the receptacle 40. The receptacle 40 is connected to a demotion hose 55, which connects the proximal end demotion hole 59 located at the bottom of the receptacle 40. The demotion hose 55 has on its journey occlusion valve 57 to allow the drainage of fluid into the receptacle housed 40. The extreme ends of the overflow hose 54 and hose 55 demotion may result in a waste receptacle (not shown), which can be a plastic bag.

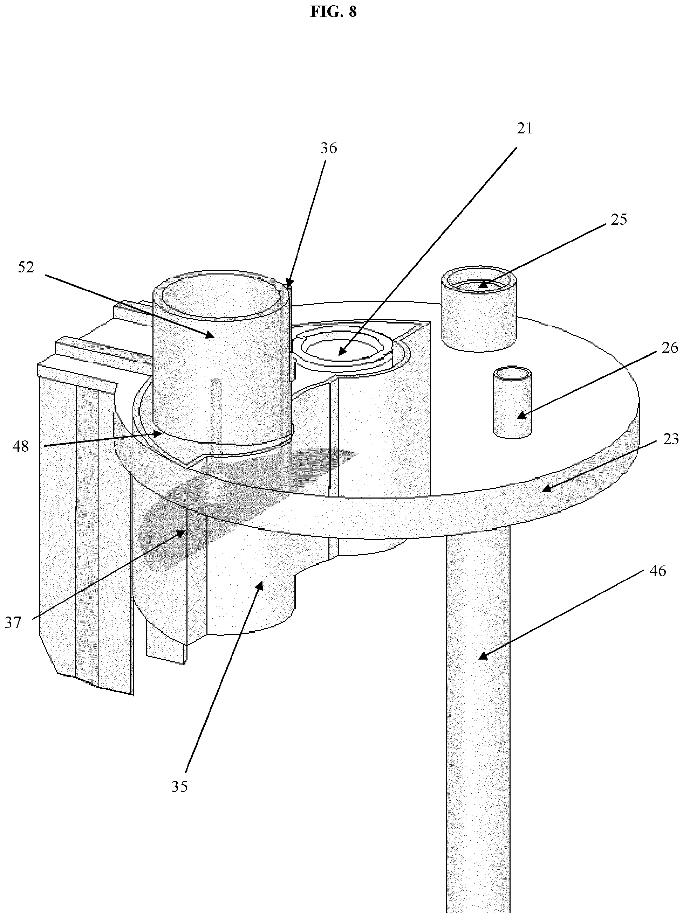

Figure 8 shows the inner vessel 35 coupled to the bottom of the top 23. The inner vessel is anchored to supports having the top 23 on the underside. The inner vessel 35 can be any size smaller than the size of the receptacle 40. Within the inner receptacle 35 is a float 37 which is shaped very similar to the inner vessel 35, but with a smaller size, fitting inside it. The float has a cylindrical extension 52 which fits into the hole 20 of the top 23. Thus, when the fluid inside the inner vessel 35 down evaporation, the float allows filling and when filling the inner vessel 35, the float 37 cuts the inflow of liquid. It should be understood that for a person skilled in the art, any other control system liquid flow can be used. Additionally, the inner receptacle 35 has a humidifying hose 38.

Figure 9 shows the stabilization mechanism of the liquid level. This comprises a stabilizer level 33, which moves axially along the rotational axis 30. The displacement of the stabilizer level 33 is accomplished by turning the enlargement 29 located at the upper end of the rotating shaft 30. The upper end of shaft 30 passes through the cover 23 through the hole 61, which additionally lets you stabilize on its longitudinal axis. Similarly, the lower end is inserted into the anchor 32 of the bottom of the receptacle 40 (see Figure 2).

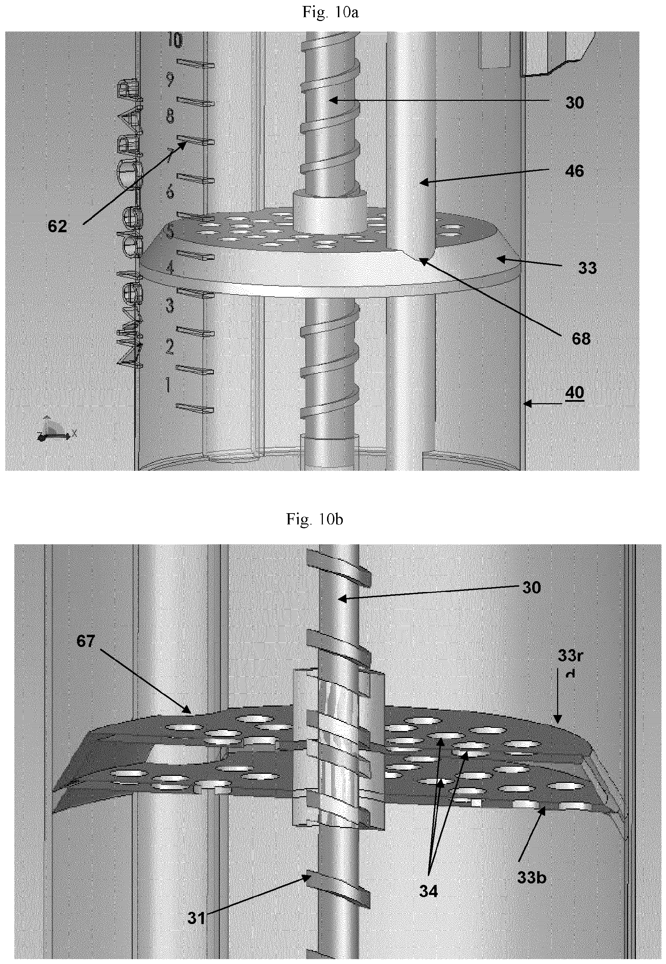

In the embodiment of the present disclosure illustrated in Figure 10, the stabilizer level 33 is formed by one or more perforated surfaces joined together (see Figure 10b). In the embodiment shown in this figure, using two drilling platforms 33a and 33b, in which the upper perforated surface 33a is the same diameter or slightly less than the internal diameter of the receptacle 40. The outer edge of the upper platform 33rd touches the wall of the receptacle 40 on which is printed or pasted 62 scale indicator of the level of CPAP. The perforated upper surface has multiple holes 33rd may be hexagonal or circular, in ways different from those shown by this figure, the diameter can vary from 0.1 mm to 7 mm. The underside of the upper perforated surface has several slots 33rd insert that allows the anchoring of the lower perforated platform between the grooves 33b, allowing them together. The platform also perforated bottom 33b has multiple holes 34 may be hexagonal or circular and whose diameter can also vary from 0.1 mm to 7 mm.

When assembled the two drilling platforms 33a and 33b, two holes are not addressed. Both the upper perforated platform 33a and 33b below the perforated platform, have a hole that has an internal thread, which is screwed to the rotating shaft 31 of screw 30. This means that when you rotate the rotating shaft 30, the stabilizer level 33 moves over the axial length of the rotary axis 30, essentially by a screw system. The direction of rotation of the shaft 30 will determine the direction of movement of the stabilizer level 33. In this mode, the overflow pipe and pressurization 44 and absorber 46 stabilize the movement of drilling rigs level stabilizer during simultaneous movement of the rotating shaft 30.

In the embodiment shown in Figure 10b, 33b lower perforated platform features multiple rows on its upper surface, which serve to direct the liquid into the holes of the upper platform 33a.

In another preferred embodiment of the disclosure the platforms 33a and 33b forming the stabilizer level, they have a hole 67 (shown in Figures 9 and 10) for the passage of the overflow pipe and pressurized internal diameter 44 may be slightly higher than the outside diameter of tube 44 and a hole 68 for passage of absorber 46, whose internal diameter may be slightly higher than the outside diameter of tube 46. In this embodiment of the invention, the overflow pipe and pressurization 44 and absorber 46 stabilize the movement of drilling rigs level stabilizer during simultaneous movement anchored on the rotating shaft 30.

### Illustrative Example:

It uses a preferred embodiment of the disclosure, including the elements described for Figure 5. To set a specific pressure installing a source of liquid above the level of the device, maintaining the occlusion valve hose 53 of the 52 closed level rise. At this point fill hose inner receptacle 52, fluid will flow to the inner vessel 35 of humidification and heating. This fluid will suspend the float 37 on the liquid, which reach the desired level to occlude the orifice 20 of the top 23, closing off the liquid. Then, connect the resistor 36 to a conventional power source.

Then you connect an oxygen source (not shown) conventional connector hospital gas source 21 of the top 23. Then connect the end of the vent pipe 6a is connected to the prongs to the hole 48 of the top 23. The other end of the prongs is connected to a vent pipe that connects to 6b hole 25 of the top 23.

Occlusion valve hose 57 demotion 55 must be closed. After opening the occlusion valve hose 53 increase level 51, and allowed to fill the receptacle 40 until the liquid level 42 to match the level of CPAP to be expressed in the brand providing the scale 62. This level can be adjusted easily, stable and secure an exact position or intermediate.

At that time you open the gas supply to the desired level, producing a bubble in the heating and humidifying receptacle 35 and to a lesser extent, into the hole 47 of the bubble tube 46. Then proceed to place a conventional face mask 7, or 8 in the nasal prongs in the patient's nostrils.

If you want to increase the dose delivered CPAP should reopen the occlusion valve hose 53 of the 51 level increased and allowed to fill the receptacle 40 until the liquid level 42 to match the level of CPAP that wish to provide.

If you want to decrease the level of CPAP given, it should then open the valve closure 57 of the hose 55 demotion until the liquid level 42 to match the level of CPAP to be supplied.

If you want to mobilize the patient to maintain the supply of CPAP, connects the front end of the resistor 36 to a portable electric source. Later operated by rotating the rotating shaft 30 at its upper end and outer 29, so that the stabilizer level 33 is moved until the outer edge of the stabilizer to match the level 33 mark of the scale 62 to the previously adjusted the fluid level 42. When they increase or decrease the dose of CPAP delivered by increasing or decreasing the level of liquid 42 within the receptacle 40, the stabilizer level 33 should be re-positioned by rotating the rotating shaft anchor 30.

Finally, for a better understanding of the cases in which the present disclosure can be applied, generally referred to a pediatric guide officers in addition to illustrate the common practices used to meet or newborn infants with respiratory problems. It describes clinical examples with specific values of liquid volume to change the pressure in a patient with a particular clinical situation. Some of these guidelines are:
- 2005 American Heart Association (AHA) Cardiopulmonary Resuscitation Guidelines for (CPR) and Emergency Cardiovascular Care (ECC) of Pediatric and Neonatal Patients: Neonatal Resuscitation Guidelines. [Pediatrics Official Journal of the American Academy of Pediatrics, August 28, 2006]
- Advances in neonatal resuscitation: Supporting transition. [Colin J. Morley and Peter G. Davis, 2008]
- A randomized, controlled trial Comparing two Different continuous positive airway pressure systems for the Extremely Successful extubation of low birth weight infants. [Pediatrics official journal of the American Academy of Pediatrics, May 23, 2007].

## Claims

1. A device for controlling pressure level during respiratory treatment, said device regulating the pressure delivered to the patient through a respiratory tool connected to a gas source, wherein said device comprises:
- a receptacle (40) containing a liquid,
- a bubble tube (46) having one end inserted into the liquid in the receptacle (40) located at a fixed level, and another end is connected to the respiratory tool;
- at least one hose (55, 50) for adjusting the liquid level in the receptacle (40) in order to adjust the pressure level;
- a stabilization mechanism for stabilizing the liquid level, wherein the stabilization mechanism comprises:
o a receptacle lid (23);
o a shaft (30) oriented along a longitudinal axis of the receptacle (40), the shaft (30) having one end connected to the receptacle lid (23) and the other end connected to a bottom of the receptacle (40), and
o at least one platform (33a, 33b) configured to move along the longitudinal axis.

2. The device of claim 1, further comprising a second receptacle (35) configured to humidify and heat the gas.

3. The device of claim 2, wherein the second receptacle (35) is found incorporated in the device.

4. The device of any of the preceding claims, wherein the device comprises liquid input and output hoses (50, 55) for adjusting the liquid level in the receptacle (40).

5. The device of any of claims 1 to 4, wherein the receptacle lid (23) having a hole for allowing the liquid to vent.

6. The device of claim 1, where the shaft (30) comprises a screw (30) and the at least one platform (33a, 33b) comprises an internal hole (34) configured to engage the screw (30), wherein the at least one platform (33a, 33b) moves along the longitudinal axis via the screw (30).

7. The device of claim 1, wherein the platform (33a, 33b) has holes (34).

8. The device of claim 7, wherein the geometric shape of the hole (34) is circular.

9. The device of claim 1, wherein the stabilizing mechanism comprises two platforms (33a, 33b) each having holes (34).

10. The device of claim 7, wherein the platform (33a, 33b) has an upper surface and one or more grooves on the upper surface, wherein the one or more grooves are configured to direct the liquid towards the holes in the platform (33a, 33b).

11. The device of claims any of claims 1 to 10, wherein the container (40), the bubble tube (46), and the at least one hose (50, 55) are arranged in a kit.

## Patentansprüche

1. Vorrichtung zum Steuern eines Druckpegels während einer Behandlung von Atembeschwerden, wobei die Vorrichtung den Druck regelt, der durch ein Atemgerät, das mit einer Gasquelle verbunden ist, zu einem Patienten geliefert wird, wobei die Vorrichtung umfasst:
- ein Behälter (40), das eine Flüssigkeit enthält,
- ein Blubberrohr (46), das mit einem Ende, das auf einer bestimmten Höhe fixiert ist, in die Flüssigkeit in dem Behälter (40) getaucht ist und das mit einem anderen Ende mit dem Atemgerät verbunden ist;
- mindestens einen Schlauch (55, 50) zum Anpassen des Flüssigkeitspegels in dem Behälter (40), um den Druckpegel anzupassen;
- einen Stabilisierungsmechanismus zum Stabilisieren des Flüssigkeitspegels, wobei der Stabilisierungsmechanismus umfasst:
∘ einen Behälterdeckel (23);
∘ einen Schaft (30), der entlang einer Längsachse des Behälters (40) ausgerichtet ist, wobei der Schaft (30) an einem Ende mit dem Behälterdeckel (23) verbunden ist und an dem anderen Ende mit einem Boden des Behälters (40) verbunden ist, und
∘ mindestens eine Plattform (33a, 33b), die dafür ausgelegt ist, sich entlang der Längsachse zu bewegen.

2. Vorrichtung nach Anspruch 1, ferner einen zweiten Behälter (35) umfassend, der dafür ausgelegt ist, das Gas zu befeuchten und zu erwärmen.

3. Vorrichtung nach Anspruch 2, wobei der zweite Behälter (35) in die Vorrichtung integriert ist.

4. Vorrichtung nach einem der vorangehenden Ansprüche, wobei die Vorrichtung Flüssigkeitszuleitungs- und -ableitungsschläuche (50, 55) zum Anpassen des Flüssigkeitspegels in dem Behälter (40) umfasst.

5. Vorrichtung nach einem der Ansprüche 1 bis 4, wobei der Behälterdeckel (23) ein Loch aufweist, um die Flüssigkeit ablassen zu können.

6. Vorrichtung nach Anspruch 1, wobei der Schaft (30) eine Schnecke (30) umfasst und die mindestens eine Plattform (33a, 33b) ein Innenloch (34) umfasst, das dafür ausgelegt ist, in die Schnecke (30) einzugreifen, wobei sich die mindestens eine Plattform (33a, 33b) über die Schnecke (30) entlang der Längsachse bewegt.

7. Vorrichtung nach Anspruch 1, wobei die Plattform (33a, 33b) Löcher (34) aufweist.

8. Vorrichtung nach Anspruch 7, wobei die geometrische Form des Loches (34) kreisrund ist.

9. Vorrichtung nach Anspruch 1, wobei der Stabilisierungsmechanismus zwei Plattformen (33a, 33b) umfasst, die jeweils Löcher (34) aufweisen.

10. Vorrichtung nach Anspruch 7, wobei die Plattform (33a, 33b) eine Oberseite und eine oder mehrere Rillen in der Oberseite aufweist, wobei die eine oder die mehreren Rillen dafür ausgelegt sind, die Flüssigkeit zu den Löchern in der Plattform (33a, 33b) zu lenken.

11. Vorrichtung nach einem der Ansprüche 1 bis 10, worin der Behälter (40), das Blubberrohr (46), und der mindestens eine Schlauch (50, 55) in einem Kit angeordnet sind.

## Revendications

1. Un dispositif pour contrôler un niveau de pression durant un traitement respiratoire, ledit dispositif régulant la pression délivrée au patient par l'intermédiaire d'un outil respiratoire relié à une source de gaz, ledit dispositif comprenant :
- un récipient (40) contenant un liquide,
- un tube à bulle (46) ayant une extrémité insérée dans le liquide dans le réceptacle (40) située à un niveau fixe, et une autre extrémité est reliée à l'outil respiratoire ;
- au moins un tuyau (55, 50) pour régler le niveau de liquide dans le récipient (40) afin de régler le niveau de pression ;
- un mécanisme de stabilisation pour stabiliser le niveau de liquide, le mécanisme de stabilisation comprenant :
∘ un couvercle (23) de réceptacle ;
∘ un arbre (30) orienté selon un axe longitudinal du réceptacle (40), l'arbre (30) ayant une extrémité connectée au couvercle de réceptacle (23) et l'autre extrémité connectée à un fond du réceptacle (40), et
∘ au moins une plate-forme (33a, 33b) configurée pour se déplacer le long de l'axe longitudinal.

2. Le dispositif selon la revendication 1, comprenant en outre un deuxième réceptacle (35) configuré pour humidifier et chauffer le gaz.

3. Le dispositif selon la revendication 2, dans lequel le deuxième réceptacle (35) est incorporé dans le dispositif.

4. Le dispositif selon l'une quelconque des revendications précédentes, dans lequel le dispositif comprend des tuyaux (50, 55) d'entrée et de sortie de liquide pour régler le niveau de liquide dans le réceptacle (40).

5. Le dispositif selon l'une quelconque des revendications 1 à 4, dans lequel le couvercle de réceptacle (23) a un trou permettant de laisser déborder le liquide.

6. Le dispositif selon la revendication 1, où l'arbre (30) comprend une vis (30) et ladite au moins une plate-forme (33a, 33b) comprend un trou interne (34) configuré pour venir en prise avec la vis (30), dans lequel ladite au moins une plate-forme (33a, 33b) se déplaçant le long de l'axe longitudinal via la vis (30).

7. Le dispositif selon la revendication 1, dans lequel la plate-forme (33a, 33b) a des trous (34).

8. Le dispositif selon la revendication 7, dans lequel la forme géométrique du trou (34) est circulaire.

9. Le dispositif selon la revendication 1, dans lequel le mécanisme de stabilisation comprend deux plates-formes (33a, 33b) présentant chacune des trous (34).

10. Le dispositif selon la revendication 7, dans lequel la plate-forme (33a, 33b) a une surface supérieure et une ou plusieurs rainures sur la surface supérieure, dans lequel lesdites une ou plusieurs rainures étant configurées pour diriger le liquide vers les trous aménagés dans la plate-forme (33a, 33b).

11. Le dispositif selon l'une quelconque des revendications 1 à 10, dans lequel le récipient (40), le tube à bulle (46) et ledit au moins un tuyau (50, 55) sont agencés en un kit.
